# EUROPEAN PATENT APPLICATION

(11) **EP 2 749 259 A1**
(43) Date of publication of application: **02.07.2014**
(21) Application number: 12824980.2
(22) Date of filing: 22.08.2012
(51) Int. Cl.: A61F 7/00

(54) **FAT-REDUCTION WEIGHT-LOSS METHOD AND WEIGHT-LOSS INSTRUMENT USING SAME**

(30) Priority: 23.08.2011 CN 201110251389
(71) Applicant: Guangzhou Beco Electronic Technology Co., Ltd, Guangzhou, Guangdong 510000 (CN)
(72) Inventor: CHEN, Defeng, Guangzhou Guangdong 510000 (CN)
(74) Representative: Delorme, Nicolas
(86) International application number: PCT/CN2012/080446
(87) International publication number: WO 2013/026393

(57) **Abstract**

A fat-reduction weight-loss method, comprising: firstly, locally heating a site in need of fat-reduction under 37 to 60°C until the blood and muscles separate from the fat; then, locally freezing the site in need of fat-reduction under -10 to 5°C until the fat solidifies. Also disclosed is a weight-loss instrument using the fat-reduction weight-loss method, comprising: an input unit (A) for receiving a user operation and outputting a signal representing the user operation to a control unit (B); a control unit (B) for outputting a heating signal or a freezing signal to a heating and freezing unit (C) according to the received signal representing the user operation; and a heating and freezing unit (C) for receiving the heating signal or freezing signal of the control unit (B) and conducting the heating or freezing operation. The fat-reduction weight-loss method at first locally heats the site in need of fat-reduction to separate the blood and muscles from the fat at the site in need of fat-reduction, then instantaneously freezes the site in need of fat-reduction to solidify the fat, thus avoiding the harms such as tissue necrosis and the like caused by the solidification of the blood and muscles together with the fat in the freezing fat-reduction process.

## Description

### FIELD OF THE INVENTION

The present invention relates to a fat-reduction method for removing body fat and a weight-loss apparatus using the same.

### BACKGROUND OF THE INVENTION

Chinese invention patent No. 200780014203.4 and No. 200780000009.0 respectively discloses a coolsculpting device designed to effect a fat reduction. All of these devices take a merit from such a basic principle that the triglyceride in adipose will solidifies at 5°C. Thus, by sending cooling wave onto the site in need of fat-reduction through a non-invasive therapeutic device, the triglyceride can be solidified through freezing. Because solidification of the triglyceride can result in it's prematurely aging, which cause the triglyceride be mildly cleansed and excreted through normal metabolism, the fat layer can be reduced and thereby a local fat reduction is effected. However, such a fat reduction method has some drawbacks which are mainly as follows: on one hand, the heavy-degree frostbite of skin epidermal tissue will occur if the skin is under an environment of 5°C for a long time; on the other hand, because majority of the fat in the blood vessel is in fusion with the blood and even much of fat are surroundingly attached to the blood vessels, the blood vessels are prone to be simultaneously frozen to solidify during freezing of the fat, which will seriously damage the human body.

To overcome one of the above drawbacks of easily producing the frostbite of epidermal tissue in the current coolsculpting fat reduction technique, Chinese invention patent No.200780000012.2 and No.200780001010.5 respectively discloses an antifreezing agent configured to be applied in coolsculpting fat reduction. The usage of such antifreezing agent can reduce effectively the frostbite of skin epidermal tissue. However, it still can not solve the problem of simultaneous solidification of the blood vessels and the fat.

### SUMMARY OF THE INVENTION

An object of the invention is to provide a fat-reduction weight-loss method capable of avoiding frostbite of tissues, such as vascular tissue and skin tissue.

A further object of the invention is to provide a weight loss apparatus applying said fat-reduction weight-loss method.

According to one aspect of the invention, it provides a fat-reduction weight-loss method, comprising: firstly, locally heating a site in need of fat-reduction under 37 °C to 60 °C until the blood and muscles separate with the fat; then, locally freezing the site in need of fat-reduction under -10°C to 5 °C until the fat solidifies.

According to the fat-reduction weight-loss method of the invention, the local heating to the site in need of fat-reduction could accelerate blood circulation at the site in need of fat-reduction and induce the blood and the muscles to separate with the fat. Then, the instant freezing to the site in need of fat-reduction could induce solidification of the fat so as to avoid injure of tissue death damage caused by the simultaneous solidification of the blood and the muscles during the coolsculpting of the fat. The solidified fat will prematurely age and be absorbed or excreted through human metabolism. A microscope can be used for observing the separation of the blood and the muscles with the fat as well as solidification of the fat.

In some embodiments, after locally heating a the site in need of fat-reduction under 37 °C to 60 °C, a pause may be taken for about 1 minute followed by locally freezing the site in need of fat-reduction under -10°C to 5 °C . The pause results in slightly decreasing epidermal temperature of human body and performing buffer function for the follow-up instant freezing.

In some embodiments, a step of applying antifreezing agent or laying an antifreezing film onto the site in need of fat-reduction may be performed prior to locally heating the site in need of fat-reduction under 37 °C to 60 °C. The antifreezing agent could be classified into freezing point reduction type and surfactants type. The freezing point reduction type antifreezing agent includes low-carbon alcohols, diatomic alcohols (polypropylene glycol and polyethylene glycol) and amide, etc. The surfactants type antifreezing agent, which brings the material forming a hydrophobic absorbing layer on the surface, generally includes acid amine salt of phosphate, alkylamine, fatty acid amide, organic acid ester, alkyl succimide, etc. This results in protecting the epidermis of human body from heating or freezing damage.

In some embodiments, sucking the site in need of fat-reduction to form wrinkles, or alternatively, firstly applying antifreezing agent or laying an antifreezing film onto the site in need of fat-reduction and then sucking the site in need of fat-reduction to form wrinkles are performed before locally heating the site in need of fat-reduction. This results in blocking the blood circulation between the site in need of fat-reduction and other regions of human body, which allows limiting the heating and the freezing only on the site in need of fat-reduction. Thereby it decreases heating/freezing time and enhancing the fat-reducing effect.

In some embodiments, the locally heating time may be 5 ∼ 40 minutes and locally freezing time may be 40 ∼ 120 minutes. Generally, temperature and time of locally heating and locally freezing are set up according to the environment temperature and thickness of the fat layer.

According to another aspect of the invention, it provides a weight-loss apparatus applying said fat-reduction weight-loss method. The apparatus comprises an input unit, a control unit and a heating/freezing unit, in which,
- the input unit is configured to receive the user's operation and output a signal representing the user's operation to the control unit;
- the control unit is configured to output a heating signal or a freezing signal to the heating/freezing unit according to the received signal representing the user's operation;
- the heating/freezing unit is configured to receive the heating signal of the control unit to conduct the heating operation, or alternatively to receive the freezing signal to conduct the freezing operation.

The weight-loss apparatus of the invention allows a complete separation of blood and fat by heating the skin and avoids injure of tissue death damage caused by the simultaneous solidification of the blood during the coolsculpting of the fat.

In some embodiments, the weight-loss apparatus further includes a mainframe and a working head. The input unit is disposed on the surface of the working head. The control unit is disposed inside the mainframe and the working head. The heating/freezing unit is disposed in the working head. The working head has a cavity provided with an opening at the lower end thereof and an air tap at the upper end thereof. The air tap is connected to a negative pressure pump disposed in the mainframe through an air pipe. The opening of the cavity bears against the skin. A motor in the mainframe drives the negative pressure pump to suck the skin close to the cavity into the inner space of the cavity. This results in blocking the blood circulation between the site in need of fat-reduction and other regions of human body, which allows limiting the heating and the freezing only on the site in need of fat-reduction. Thereby it decreases heating/freezing time and enhancing the fat-reducing effect.

In some embodiments, the heating/freezing unit may be disposed at the left and right side of the cavity of the working head. This results in a uniform heating or freezing at the site in need of fat-reduction so as to protect the skin from damage caused by concentrated heating.

In some embodiments, the working head may be provided with a handle. This results in facilitating of user operation.

In some embodiments, the control unit may be a microcomputer and the heating/freezing unit may be a semiconductor cooler. The semiconductor cooler includes a heat end designed to heat the skin and a cold end designed to cool the skin. The semiconductor cooler arranged in the cavity of the working head makes the weight-loss apparatus safe and environmental friendly because the cooler does not have a sliding part and any contamination freezing agents.

In some embodiments, a radiator is provided at the back of the semiconductor cooler and the mainframe is provided with therein a radiating water tank having a water inlet and a water outlet connected with a water pipe. The water pipe coils round at the back of the radiator and the radiating water tank is provided with a water pump therein such that water in the radiating water tank and in the water pipe creates a circulation circuit. The water in the radiating water tank out of the water outlet flows through the water pipe and absorbs heat of the radiator. Then it transfers the heat to the air through the pipe at the water inlet and lastly flows into the radiating water tank. The radiator is mainly used for decreasing the heat of the heat end of the semiconductor cooler and thereby decreasing the cold end temperature to achieve the refrigeration. This results in accelerating of the cooling rate of the semiconductor cooler and allows it to reach a lower temperature.

In some embodiments, the cavity may be made from silica gel or soft materials. As a result, the cavity can be tightly against the human skin without interspaces, which favors the suction of the skin into the cavity.

In some embodiments, the cavity may be transparent. As a result, the changes of the skin in the cavity can be observed through the cavity so as to turn off the weight-loss apparatus immediately in case of unexpected circumstances.

In some embodiments, a temperature sensor may be provided in the cavity. The temperature sensor can be used for measuring the temperature change of the working area (namely, the semiconductor cooler and the skin). And controlling of the fat-reduction process can be achieved according to the temperature change. This allows avoiding heating or cooling for too long or too short.

In some embodiments, the cavity is provided with a LED lamp on the top thereof, which comprises red light, green light, blue light and other different colored lights. The red LED light is used to increase blood oxygen content for the skin in the cavity and promote blood circulation and metabolism of the skin in the cavity so as to enhance the effect of the weight-loss apparatus. The green LED light is turned on during freezing of the skin to effect anti-inflammatory so as to protect effectively the skin epidermis from freezing damage.

### BRIEF DESCRIPTION OF THE DRAWINGS

Fig.1 is a structural principle diagram of the weight-loss apparatus according to one embodiment of the invention.
Fig.2 is a structural schematic view of the weight-loss apparatus according to one embodiment of the invention.
Fig.3 is a partial cross-sectional view of the working head in Fig.2.
Fig.4 is another partial cross-sectional view of the working head in Fig.2.
Fig.5 is an exploded structural view of the working head in Fig.2.

### DETAILED DESCRIPTION OF PREFERRED EMBODIMENT

The present invention can be further understood with reference to the related appended drawings and the following exemplary embodiments.

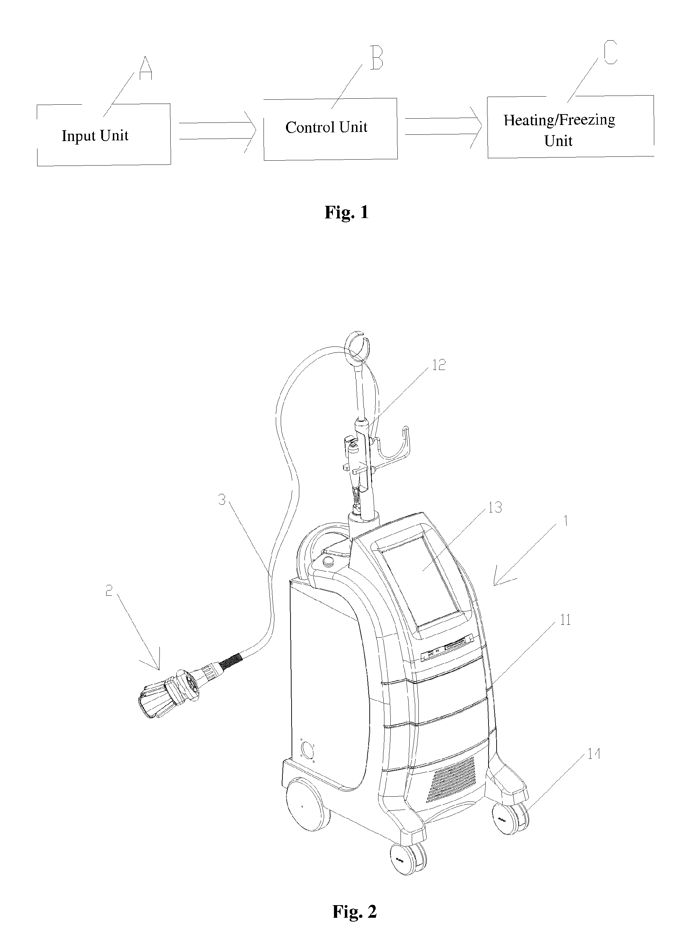

Fig.1 schematically shows a structural principle diagram of the weight-loss apparatus according to one embodiment of the invention.

As shown in Fig. 1, a weight-loss apparatus comprises an input unit A, a control unit B and a heating/freezing unit C, in which
the input unit A is configured to receive the user's operation and output a signal representing the user's operation to the control unit B;
the control unit B is configured to output a heating signal or a freezing signal to a heating/freezing unit C according to the received signal representing the user's operation;
the heating/freezing unit C is configured to receive the heating signal of the control unit B to conduct the heating operation, or alternatively to receive the freezing signal to conduct the freezing operation.

Fig.2 schematically shows the structure of the weight-loss apparatus including one embodiment of the functional unit shown in Fig.1.

As shown in Fig.2, a weight-loss apparatus comprises a mainframe 1, a working head 2 and a bourdon tube 3. The input unit A is provided on the surface of the working head 2. The control unit B is a microcomputer which may be provided in the mainframe 1 and the working head 2. The heating/freezing unit C is provided in the working head 2.

The mainframe 1 comprises a body 11 and a support frame 12 mounted on the body 11 and arranged to place the working head 2. The body 11 has a display screen 13 on the surface thereof and four wheels 14 thereunder. The body 11 also has set up therein a negative pressure pump, a motor for driving the negative pressure pump and a radiating water tank. The radiating water tank has a water inlet and a water outlet connected with a water pipe 28. The radiating water tank is provided with a water pump such that water in the radiating water tank and the water pipe 28 creates a circulation circuit when the water pump is started.

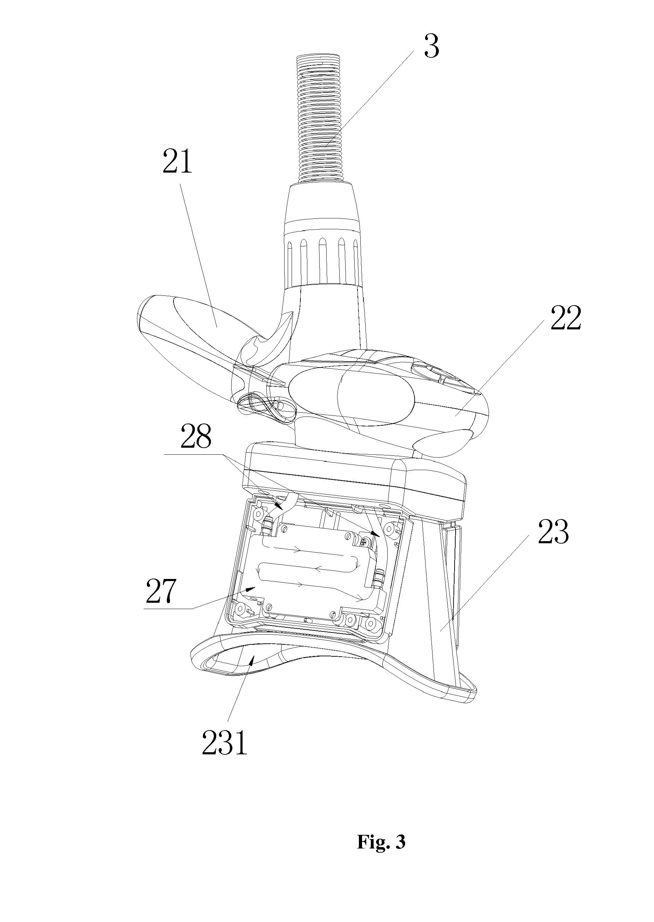

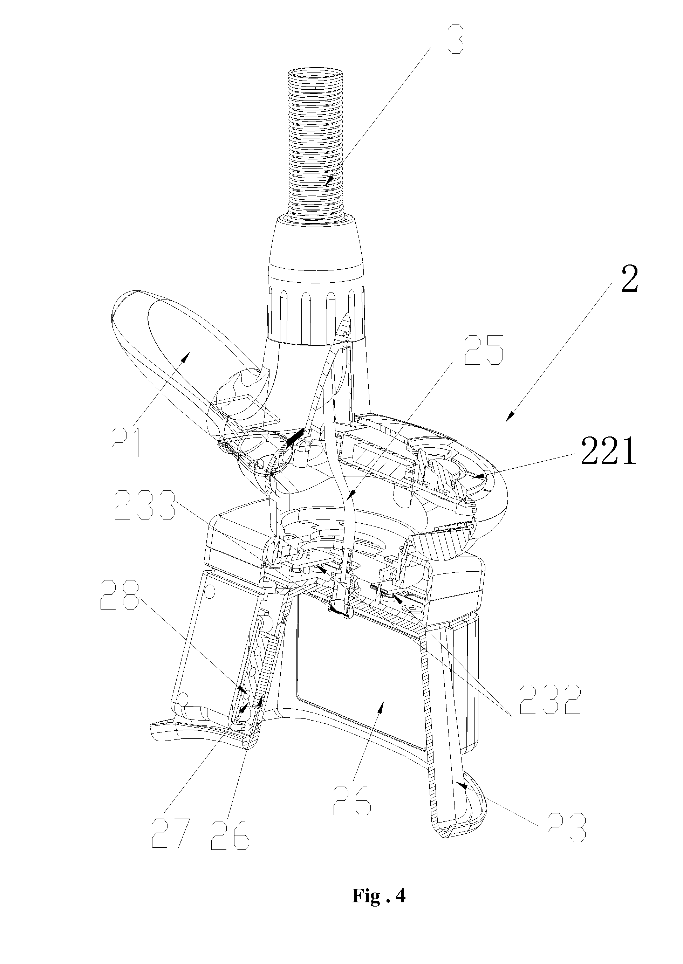

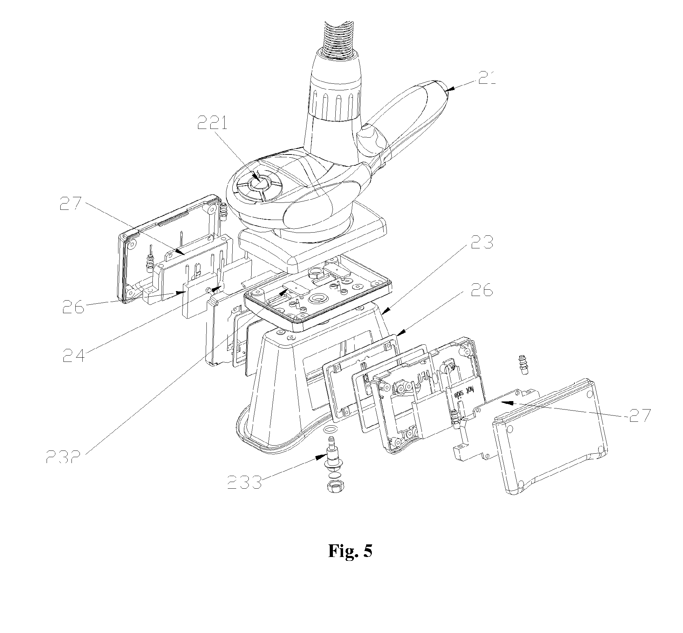

As shown in Fig.3, Fig.4 and Fig.5, the working head 2 is provided with a handle 21, a main body 22 and a cavity 23 arranged to suck the skin. The handle 21 is secured to the back end of the body 22. The cavity 23 is disposed beneath the main body 22.

The input unit A is mounted on the surface of the working head 2 and it is particularly an operation keyboard 221 disposed on the surface of the main body 22. Moreover, the control unit B is disposed in the main body 22.

The cavity 23 is a transparent object made from silica gel or soft materials. The bottom of the cavity 23 opens wide to form an opening 231. On the top of the cavity 23 is provided with a LED lamp panel 232 on which mounted LED light beads with different colors such as red, green, and blue. Inside the cavity 23 is provided with a temperature sensor 24.

On the top of the cavity 23 is further provided with an air tap 233. An air pipe 25 is communicated at one end thereof with the air tap 233 to connect the cavity and the air pipe 25. The other end of the air pipe 25 passes through the main body 22 of the working head 2 and is connected with the negative pressure pump disposed in the mainframe 1.

The heating/freezing unit C is a semiconductor cooler 26 which is disposed at the left and right side of the cavity 23. The semiconductor cooler 26 includes a heat end and a cold end in which the heat end is arranged to heat the skin and the cold end is arranged to cool the skin.

The semiconductor cooler 26 is provided with a radiator 27 at the back side thereof and a water pipe coils round at the back side of the radiator 27. The water pipe 28 is communicated at one end thereof with the water inlet of the radiating water tank in the mainframe 1 through the main body 22 of the working head 2 and at the other end thereof with the water outlet of the radiating water tank in the mainframe 1 through the main body 22 of the working head 2.

The bourdon tube 3 is connected at one end thereof with the mainframe 1 and at the other end thereof with the working head 2. The air pipe 25, the water pipe 28 and other data wires connecting the mainframe 1 and the working head 2 are all packaged in the bourdon tube 3.

When in operation, one first applies antifreezing agent or lays an antifreezing film onto the site of skin in need of fat-reduction. Then allow the opening 231 of the cavity 23 of the working head 2 to bear against the skin so as to form a closure space with the skin. The antifreezing agent is alkylamine in this embodiment. In other embodiments, however, the antifreezing agent may be low-carbon alcohols, diatomic alcohols (polypropylene glycol and polyethylene glycol), amide, acid amine salt of phosphate, alkylamine, fatty acid amide, organic acid ester or alkyl succimide.

Next, turn on the mainframe 1 and allow the motor to drive the negative pressure pump. At this time, the circulation system of the radiator 27 and the radiating water tank is shut off. The air in the closure space formed between the cavity 23 and the skin is sucked out through the air pipe 25 by the negative pressure pump. Under the negative pressure, the skin is sucked into the cavity 23 and forms wrinkles. Thus, the blood circulation between the sucked skin and the other region of skin is cut off.

Pressing the heating button of the operation keyboard 221 will make the control unit B send out a heating signal. At this time, the skin is heated by the semiconductor cooler 26 and the red LED light. The temperature data in the cavity 23 is transmitted to the control unit by the temperature sensor 24 and then displayed on the display screen 13. The temperature in the cavity 23 is controlled as 37∼60°C(for example 45°C). Continuously heating of the skin accelerates the blood circulation of the skin in the cavity 23. Heating of the skin continues for 5∼40 minutes (for example 10 minutes) until the blood and the muscles separate with the fat. Such an internal change situation of the skin can be detected by a device like B mode ultrasound device mounted in the cavity 23. This device transmits a signal representing the internal change situation of the skin to the control unit and the signal is displayed on the display screen 13. Thus, the internal change situation of the skin can be observed by the display screen 13 so as to facilitate judging whether the blood and the muscles are separated completely with the fat.

Upon the above heating step is finished, a pause for about 1 minute is taken. Then, the circulation system of the radiator 27 and the radiating water tank as well as the LED lamp are turn on to locally freeze the site in need of fat-reduction under -10 °C to 5°C (e.g. at 5°C) for 40∼120min (e.g.45min) until the fat solidifies. Finally, the mainframe 1 is turned off to let air enter the cavity 23 such that the skin recoveries. The whole fat-reduction process is accomplished.

## Claims

1. A fat-reduction weight-loss method, comprising:
firstly, locally heating a the site in need of fat-reduction under 37°C to 60°C until the blood and muscles separate with the fat;
then, locally freezing the site in need of fat-reduction under -10°C to 5°C until the fat solidifies.

2. The fat-reduction weight-loss method according to claim 1, **characterized in that** it comprises: firstly locally heating the site in need of fat-reduction under 37°C to 60°C; taking a pause for about 1 minute; and then locally freezing the site in need of fat-reduction under -10°C to 5°C .

3. The fat-reduction weight-loss method according to claim 2, **characterized in that** it comprises: firstly applying antifreezing agent or laying an antifreezing film onto the site in need of fat-reduction; locally heating the site in need of fat-reduction under 37°C to 60°C; taking a pause for about 1 minute; and then locally freezing the site in need of fat-reduction under -10°C to 5°C .

4. The fat-reduction weight-loss method according to claim 3, **characterized in that** it comprises: firstly applying antifreezing agent or laying an antifreezing film onto the site in need of fat-reduction; sucking the site in need of fat-reduction to wrinkle it; locally heating the site in need of fat-reduction under 37 °C to 60 °C ; taking a pause for about 1 minute; and then locally freezing the site in need of fat-reduction under -10°C to 5°C .

5. A weight-loss apparatus applying the fat-reduction weight-loss method according to any of claims 1 to 4, **characterized in that** it comprises a mainframe and a working head connected to the mainframe, the working head being provided with a cavity for sucking the skin, the cavity being connected via a pipe to a negative pressure pump arranged in the mainframe, a working area of semiconductor cooler being provided at least at one side of the cavity, the working area of semiconductor cooler being able to provide heating or freezing functions according to different signals received, the semiconductor cooler in the working area having a radiator arranged on the back side thereof and the radiator being connected to a radiating water tank arranged in the mainframe, the working area of semiconductor cooler being able to heat the skin based on a heating signal input through a control unit and to instantly freeze the skin based on a freezing signal input through the control unit.

6. The weight-loss apparatus according to claim 5, **characterized in that** the cavity has LED lamps arranged on the top thereof.

7. The weight-loss apparatus according to claim 6, **characterized in that** the working head is provided with a handle.

8. The weight-loss apparatus according to claim 7, **characterized in that** the cavity is transparent and made of silica gel or soft materials.

9. The weight-loss apparatus according to claim 8, **characterized in that** the cavity is provided with a temperature sensor therein.

10. The weight-loss apparatus according to claim 9, **characterized in that** the LED lamps comprises LED lamps with red light, green light and blue light.
